# EUROPEAN PATENT APPLICATION

(11) **EP 4 230 514 A1**
(43) Date of publication of application: **23.08.2023**
(21) Application number: 21880114.0
(22) Date of filing: 12.10.2021
(51) Int. Cl.: B63B 25/16, F17C 9/00, G01N 1/00, B63B 79/30

(54) **GAS INSPECTING METHOD, AND GAS INSPECTING FACILITY**

(30) Priority: 14.10.2020 JP 2020173179
(71) Applicant: KAWASAKI JUKOGYO KABUSHIKI KAISHA, Kobe-shi, Hyogo 650-8670 (JP)
(72) Inventor: SHIMOGAKI, Takashi, Kobe-shi, Hyogo 6508670 (JP); KASHIWA, Shinichiro, Kobe-shi, Hyogo 6508670 (JP); UNNO, Shuntaro, Kobe-shi, Hyogo 6508670 (JP)
(74) Representative: Arnold & Siedsma
(86) International application number: PCT/JP2021/037759
(87) International publication number: WO 2022/080376

(57) **Abstract**

A gas inspection method according to one aspect of the present application is a gas inspection method performed in a liquefied gas carrier, the gas inspection method including: attaching a gas inspection structure including a gas inspector to between an extractor located at a combustible gas facility and a disposal line connected to an exhaust gas treatment facility, to form a gas inspection line between the combustible gas facility and the exhaust gas treatment facility; introducing an inspection target gas from the combustible gas facility to the gas inspection line; subjecting the inspection target gas, introduced to the gas inspection line, to gas inspection by the gas inspector in the gas inspection line; after the inspection target gas is subjected to the gas inspection, introducing an inert gas to the gas inspection line to discharge the inspection target gas, remaining in the gas inspection line, to the exhaust gas treatment facility through the disposal line; and after an inside of the gas inspection line is deactivated, detaching the gas inspection structure from between the extractor and the disposal line.

## Description

### Technical Field

The present application relates to a gas inspection method performed in a liquefied gas carrier and a gas inspection facility located in the liquefied gas carrier.

### Background Art

A liquefied gas carrier is a ship that carries a liquefied gas, prepared by liquefying a combustible gas, from one land terminal to another land terminal (see PTL 1). The liquefied gas carrier includes facilities (hereinafter referred to as "combustible gas facilities") such as a tank and a pipe, which contain a combustible gas. The combustible gas facility includes an extractor that extracts s gas therein. The gas extracted through the extractor is subjected to inspection (hereinafter referred to as "gas inspection") regarding properties, combustible gas concentration, and the like by a gas inspector.

### Citation List

### Patent Literature

PTL 1: Japanese Laid-Open Patent Application Publication No. 2018-203106

### Summary of Invention

### Technical Problem

Since the liquefied gas carrier includes a large number of extractors located at the combustible gas facilities, it is not realistic to provide the gas inspectors at all the extractors. Therefore, it is effective to perform the gas inspection by attaching a detachable gas inspector to each extractor according to need. However, when using such detachable gas inspector, a problem is how to perform disposal treatment of the gas (hereinafter referred to as an "inspection target gas") extracted for the gas inspection.

The present application was prepared under these circumstances, and an object of the present application is to provide a gas inspection method and a gas inspection facility, each of which, when performing gas inspection by using a detachable gas inspector in a liquefied gas carrier, can appropriately perform disposal treatment of an inspection target gas extracted for the gas inspection.

### Solution to Problem

A gas inspection method according to one aspect of the present application is a gas inspection method performed in a liquefied gas carrier. The gas inspection method includes:
attaching a gas inspection structure including a gas inspector to between an extractor located at a combustible gas facility and a disposal line connected to an exhaust gas treatment facility, to form a gas inspection line between the combustible gas facility and the exhaust gas treatment facility;
introducing an inspection target gas from the combustible gas facility to the gas inspection line;
subjecting the inspection target gas, introduced to the gas inspection line, to gas inspection by the gas inspector in the gas inspection line; after the inspection target gas is subjected to the gas inspection, introducing an inert gas to the gas inspection line to discharge the inspection target gas, remaining in the gas inspection line, to the exhaust gas treatment facility through the disposal line;
and after an inside of the gas inspection line is deactivated, detaching the gas inspection structure from between the extractor and the disposal line.

A gas inspection facility according to another aspect of the present application is a gas inspection facility located at a liquefied gas carrier. The gas inspection facility includes: an extractor located at a combustible gas facility; a disposal line connected to an exhaust gas treatment facility; and a gas inspection structure that is detachably attached to between the extractor and the disposal line and performs gas inspection of an inspection target gas extracted from the combustible gas facility.

### Advantageous Effects of Invention

The above configurations can provide a gas inspection method and a gas inspection facility, each of which, when performing gas inspection by using a detachable gas inspector in a liquefied gas carrier, can appropriately perform disposal treatment of an inspection target gas extracted for the gas inspection.

### Brief Description of Drawings

FIG. 1 is a schematic diagram of a gas inspection facility.
FIG. 2 is a flowchart showing a gas inspection method.
FIG. 3 is a schematic diagram of the gas inspection facility according to Modified Example.

### Description of Embodiments

### Gas Inspection Facility

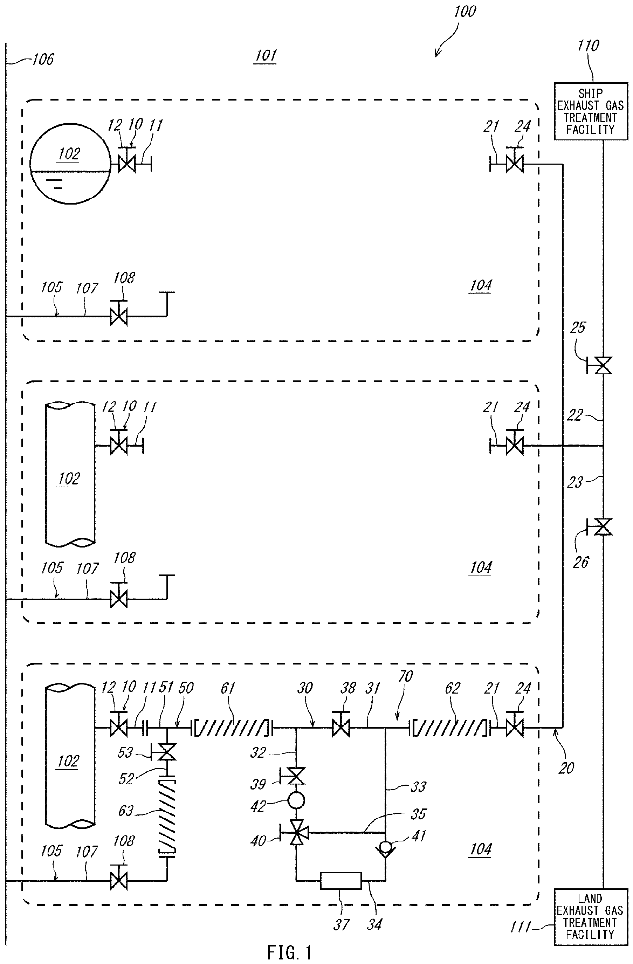

First, a gas inspection facility 100 according to an embodiment will be described. FIG. 1 is a schematic diagram of the gas inspection facility 100. The gas inspection facility 100 is located in a liquefied gas carrier 101 that carries a liquefied gas, prepared by liquefying a combustible gas, from one land terminal to another land terminal. Examples of the combustible gas include a hydrogen gas and a natural gas.

The gas inspection facility 100 includes extractors 10, a disposal line 20, a gas inspection structure 30, and an inert gas supply structure 50. Hereinafter, these components will be described in order.

### Extractor

The extractor 10 is a device that extracts a gas from a combustible gas facility 102. The combustible gas facility 102 is a facility that contains the combustible gas therein. For example, since a tank that stores the liquefied gas contains the combustible gas therein, such tank corresponds to the combustible gas facility 102. Moreover, when the combustible gas temporarily flows in a pipe through which the liquefied gas flows, not only a pipe through which the combustible gas flows but also the pipe through which the liquefied gas flows corresponds to the combustible gas facility 102. FIG. 1 shows three combustible gas facilities 102, and the extractors 10 are located for the respective combustible gas facilities 102.

Each extractor 10 of the embodiment includes an extraction line 11 and an extraction valve 12 located on the extraction line 11. In FIG. 1, one extraction valve 12 is located on each extraction line 11. However, two or more extraction valves 12 may be located on each extraction line 11. An opening degree of the extraction valve 12 may be manually adjusted, may be automatically adjusted, or may be able to be both manually and automatically adjusted. The same is true for below-described valves.

### Disposal Line

The disposal line 20 is a line connected to an exhaust gas treatment facility. The exhaust gas treatment facility includes: a ship exhaust gas treatment facility 110 located at the liquefied gas carrier 101; and a land exhaust gas treatment facility 111 located on land. The ship exhaust gas treatment facility 110 includes: a liquefied gas combustor that treats the liquefied gas; a vent mast that prevents an increase in pressure in a tank that stores the liquefied gas; and the like. The disposal line 20 of the embodiment is connected to both the ship exhaust gas treatment facility 110 and the land exhaust gas treatment facility 111. When the liquefied gas carrier 101 lies alongside the land terminal, the land exhaust gas treatment facility 111 can be connected to the disposal line 20 through a line extending from the land exhaust gas treatment facility 111.

The disposal line 20 includes connection lines 21, a ship branch line 22, and a land branch line 23. The connection lines 21 are located so as to correspond to respective work areas 104 at each of which the extractor 10 is located. Each work area 104 may be indoor or outdoor. Connection valves 24 are located on the respective connection lines 21.

The connection lines 21 are combined with each other, and the combined line is divided into the ship branch line 22 and the land branch line 23. The ship branch line 22 is a line connecting the connection lines 21 and the ship exhaust gas treatment facility 110. Moreover, the land branch line 23 is a line connecting the connection lines 21 and the land exhaust gas treatment facility 111. The connection lines 21 may not be combined with each other, and each connection line 21 may be divided into the ship branch line 22 and the land branch line 23. A ship valve 25 is located on the ship branch line 22, and a land valve 26 is located on the land branch line 23.

### Gas Inspection Structure

The gas inspection structure 30 is a unit that performs gas inspection of a gas (inspection target gas) extracted from the combustible gas facility 102 through the extractor 10. The gas inspection structure 30 can be carried and can be detachably connected to each extractor 10. In the work area 104 shown at a lowermost side in FIG. 1, the gas inspection structure 30 is connected to the extractor 10 through the inert gas supply structure 50 and a flexible pipe 61. Moreover, the gas inspection structure 30 is connected to the connection line 21 of the disposal line 20 through a flexible pipe 62. To be specific, the gas inspection structure 30 is detachably attached to between the extractor 10 and the disposal line 20.

The gas inspection structure 30 includes a direct connection line 31, an inlet line 32, an outlet line 33, a main line 34, and a bypass line 35.

The direct connection line 31 is a line connecting an inlet and outlet of the gas inspection structure 30. The inlet line 32 is a line which extends from a portion, close to the inlet, of the direct connection line 31 and into which the inspection target gas flows. The outlet line 33 is a line which is coupled to a portion, close to the outlet, of the direct connection line 31 and from which the inspection target gas flows out. The main line 34 is a line which connects the inlet line 32 and the outlet line 33 and on which a below-described gas inspector 37 is located. The bypass line 35 is a line which connects the inlet line 32 and the outlet line 33 and is located in parallel with the main line 34.

The gas inspection structure 30 further includes the gas inspector 37, a direct connection valve 38, a flow rate adjustment valve 39, a switching valve 40, a check valve 41, and a flow meter 42.

The gas inspector 37 is a device that is located on the main line 34 and inspects the inspection target gas extracted from the combustible gas facility 102. The gas inspector 37 of the embodiment is a concentration inspector that inspects a percentage of the combustible gas in the inspection target gas. However, the gas inspector 37 may be a device other than the concentration inspector. The gas inspector 37 may be detachably located on the main line 34. The direct connection valve 38 is located on the direct connection line 31 and between the portion of the direct connection line 31 from which the inlet line 32 extends and the portion of the direct connection line 31 to which the outlet line 33 is coupled.

The flow rate adjustment valve 39 is located on the inlet line 32. The flow rate adjustment valve 39 can adjust the flow rate of the inspection target gas in the inlet line 32, and therefore, can adjust the flow rate of the inspection target gas flowing into the gas inspector 37. The switching valve 40 is located at a border portion of the inlet line 32, the main line 34, and the bypass line 35. The switching valve 40 is a so-called three-way valve and can select any of the main line 34 and the bypass line 35 as a line to which the inspection target gas having flowed through the inlet line 32 is supplied.

The check valve 41 is located on the main line 34 and downstream of the gas inspector 37. The check valve 41 is a valve that prevents the inspection target gas from flowing backward to the gas inspector 37. The flow meter 42 is located on the inlet line 32 and can measure the flow rate of the inspection target gas flowing through the inlet line 32. The flow meter 42 of the embodiment is located downstream of the flow rate adjustment valve 39. However, the flow meter 42 may be located upstream of the flow rate adjustment valve 39.

### Inert Gas Supply Unit

The inert gas supply structure 50 is a unit that supplies an inert gas to between the extractor 10 and the gas inspection structure 30. Examples of the inert gas include a nitrogen gas and an argon gas. The inert gas supply structure 50 can be carried and can be detachably attached to between the extractor 10 and the gas inspection structure 30. In the embodiment, the inert gas supply structure 50 is connected to the gas inspection structure 30 through the flexible pipe 61.

The inert gas supply structure 50 includes: a direct connection line 51 connecting an inlet and outlet of the inert gas supply structure 50; a supply line 52 coupled to the direct connection line 51; and a supply valve 53 located on the supply line 52. Moreover, the supply line 52 can be detachably connected to an inert gas supplier 105 through a flexible pipe 63.

The inert gas supplier 105 is located in each work area 104. The inert gas supplier 105 is connected to an inert gas line 106 laid in the liquefied gas carrier 101. The inert gas supplier 105 can extract the inert gas in the inert gas line 106. The inert gas supplier 105 includes an inert gas supply line 107 and an inert gas supply valve 108 located on the inert gas supply line 107.

In the embodiment, the inert gas is supplied to a gas inspection line 70 by using the inert gas supply structure 50. However, the inert gas may be supplied to the gas inspection line 70 by another method. For example, the inert gas may be supplied to the gas inspection line 70 by supplying the inert gas from the inert gas supplier 105 to the combustible gas facility 102.

### Gas Inspection Method

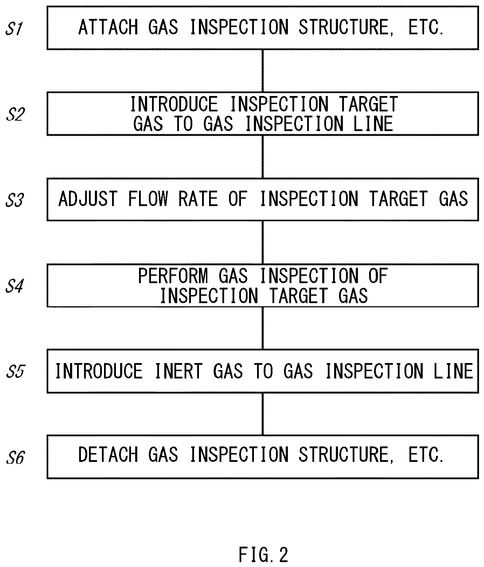

Next, the gas inspection method according to the embodiment will be described. Herein, a percentage of the combustible gas in the gas in the combustible gas facility 102 is inspected. Moreover, the gas inspection is performed in one of the work areas 104 (i.e., the work area 104 located at the lowermost side in FIG. 1). FIG. 2 is a flowchart showing the gas inspection method. The gas inspection method described below may be manually performed or may be automatically performed.

First, the gas inspection structure 30 and the inert gas supply structure 50 are attached to between the extractor 10 and the disposal line 20 (Step S1). Specifically, the gas inspection structure 30 is attached to the extractor 10 through the inert gas supply structure 50 and the flexible pipe 61. Then, the gas inspection structure 30 is attached to the disposal line 20 through the flexible pipe 62. At this time, the inert gas supply structure 50 is attached to the inert gas supplier 105 through the flexible pipe 63.

With this, the extractor 10, the inert gas supply structure 50, the flexible pipe 61, the gas inspection structure 30, the flexible pipe 62, and the disposal line 20 are connected to each other in this order from an upstream side and form the gas inspection line 70. To be specific, the gas inspection line 70 is formed between the combustible gas facility 102 and the ship exhaust gas treatment facility 110 and between the combustible gas facility 102 and the land exhaust gas treatment facility 111. When forming the gas inspection line 70, all the valves 12, 24, 25, 26, 38, 39, and 53 other than the switching valve 40 are closed.

Next, the inspection target gas is introduced from the combustible gas facility 102 to the gas inspection line 70 (Step S2). Specifically, the extraction valve 12, the direct connection valve 38, the connection valve 24, and the ship valve 25 are open. With this, the inspection target gas extracted from the combustible gas facility 102 flows through the gas inspection line 70 toward the ship exhaust gas treatment facility 110. To be specific, the inspection target gas having flowed through the gas inspection line 70 is discharged to the ship exhaust gas treatment facility 110, not the work area 104.

Next, the flow rate of the inspection target gas is adjusted (Step S3). Specifically, the direct connection valve 38 is closed, and the flow rate adjustment valve 39 is open. Then, the switching valve 40 is operated such that the inspection target gas having flowed through the inlet line 32 flows through the bypass line 35. With this, the inspection target gas having flowed into the gas inspection structure 30 flows through the bypass line 35 and flows out from the gas inspection structure 30. In this state, while confirming the flow meter 42, the flow rate adjustment valve 39 is adjusted such that the flow rate of the inspection target gas becomes a preset flow rate.

Next, the gas inspection of the inspection target gas is performed by using the gas inspector 37 of the gas inspection structure 30 (Step S4). Specifically, the switching valve 40 is operated such that the inspection target gas having flowed through the inlet line 32 flows through the main line 34. With this, the inspection target gas flows through the gas inspector 37, and at this time, the gas inspection is performed by the gas inspector 37. As described above, the flow rate of the inspection target gas is adjusted to the preset flow rate. Therefore, damage of the gas inspector 37 which is caused when a large amount of inspection target gas flows into the gas inspector 37 can be prevented. The inspection target gas having been subjected to the inspection flows through the disposal line 20 and is discharged to the ship exhaust gas treatment facility 110.

Next, after the inspection target gas is subjected to the gas inspection, the inert gas is introduced to the gas inspection line 70 (Step S5). Specifically, the extraction valve 12 is closed, and the inert gas supply valve 108 and the supply valve 53 are open. With this, the inspection target gas remaining in the gas inspection line 70 is pushed out by the inert gas to be discharged to the ship exhaust gas treatment facility 110.

In the embodiment, when introducing the inert gas to the gas inspection line 70, a percentage of the combustible gas in the gas in the gas inspection line 70 is inspected by utilizing the gas inspector 37. Then, the inert gas is introduced to the gas inspection line 70 until the percentage of the combustible gas in the gas in the gas inspection line 70 becomes a fixed percentage or less (specifically, such a percentage that there is no possibility that the gas is ignited). With this, the inside of the gas inspection line 70 is deactivated. The inert gas is also supplied to the direct connection line 31 and the bypass line 35 of the gas inspection structure 30, and the gas in these lines is also discharged to the ship exhaust gas treatment facility 110.

Finally, the gas inspection structure 30 and the inert gas supply structure 50 are detached from between the extractor 10 and the disposal line 20 (Step S6). At this time, all the valves 12, 24, 25, 26, 38, 39, and 53 other than the switching valve 40 are closed. By detaching the structures 30 and 50, part of the gas in the gas inspection structure 30, the inert gas supply structure 50, and the flexible pipes 61, 62, and 63 is discharged into the work area 104. However, since the percentage of the combustible gas in the gas discharged as above is the fixed percentage or less, the combustible gas is not ignited in the work area.

In the above gas inspection method, the gas having flowed through the gas inspection line 70 is discharged to the ship exhaust gas treatment facility 110. However, the gas having flowed through the gas inspection line 70 may be discharged to the land exhaust gas treatment facility 111 by closing the ship valve 25 and opening the land valve 26. Moreover, the gas having flowed through the gas inspection line 70 may be discharged to both the ship exhaust gas treatment facility 110 and the land exhaust gas treatment facility 111 by opening both the ship valve 25 and the land valve 26. The gas discharged to the ship exhaust gas treatment facility 110 and the land exhaust gas treatment facility 111 is appropriately treated, i.e., burned, for example.

### Modified Example

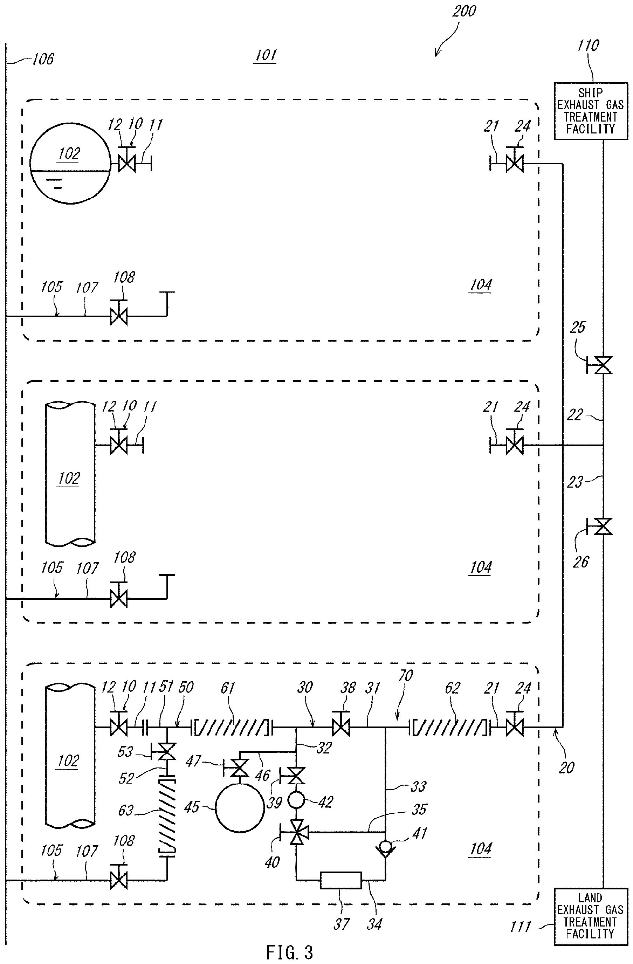

Next, a gas inspection facility 200 according to Modified Example of the above embodiment will be described. FIG. 3 is a schematic diagram of the gas inspection facility 200 according to Modified Example. The gas inspection facility 200 according to Modified Example is different in configuration from the gas inspection facility 100 according to the above embodiment in that the gas inspection structure 30 includes a sampling balloon 45. Other than the above, the gas inspection facility 200 according to Modified Example is basically the same in configuration as the gas inspection facility 100 according to the above embodiment.

The sampling balloon 45 is located upstream of the flow rate adjustment valve 39 of the inlet line 32 through a balloon line 46. A balloon valve 47 is located on the balloon line 46. The sampling balloon 45 is made of a stretchable material. The sampling balloon 45 can temporarily store the inspection target gas taken from the inlet line 32 and discharge the stored inspection target gas to the inlet line 32. A timing to store the gas in the sampling balloon 45 and a timing to discharge the gas from the sampling balloon 45 can be adjusted by opening or closing the balloon valve 47.

As above, in the gas inspection facility 200 according to Modified Example, the sampling balloon 45 can temporarily store the inspection target gas. Therefore, the gas inspection can be performed in a state where the introduction of the inspection target gas from the combustible gas facility 102 to the gas inspection line 70 is stopped. On this account, according to the gas inspection facility 200 of Modified Example, the degree of freedom of the gas inspection improves.

### Operational Advantages

As described above, a gas inspection method according to the embodiment is a gas inspection method performed in a liquefied gas carrier. The gas inspection method includes: attaching a gas inspection structure including a gas inspector to between an extractor located at a combustible gas facility and a disposal line connected to an exhaust gas treatment facility, to form a gas inspection line between the combustible gas facility and the exhaust gas treatment facility; introducing an inspection target gas from the combustible gas facility to the gas inspection line; subjecting the inspection target gas, introduced to the gas inspection line, to gas inspection by the gas inspector in the gas inspection line; after the inspection target gas is subjected to the gas inspection, introducing an inert gas to the gas inspection line to discharge the inspection target gas, remaining in the gas inspection line, to the exhaust gas treatment facility through the disposal line; and after an inside of the gas inspection line is deactivated, detaching the gas inspection structure from between the extractor and the disposal line.

According to this method, the inspection target gas used for the gas inspection and the inspection target gas remaining in the gas inspection line are discharged to the exhaust gas treatment facility. Therefore, according to the above method, the inspection target gas extracted for the gas inspection can be appropriately subjected to disposal treatment.

Moreover, in the gas inspection method according to the embodiment, when introducing the inert gas to the gas inspection line, a percentage of a combustible gas in the gas in the gas inspection line is inspected by utilizing the gas inspector, and the inert gas is introduced to the gas inspection line until the percentage of the combustible gas in the gas in the gas inspection line becomes a fixed percentage or less.

According to this method, the gas inspection structure can be detached after it is confirmed that the inspection target gas extracted for the gas inspection is appropriately subjected to the disposal treatment.

As above, the gas inspection facility according to the embodiment is a gas inspection facility located at a liquefied gas carrier. The gas inspection facility includes: an extractor located at a combustible gas facility; a disposal line connected to an exhaust gas treatment facility; and a gas inspection structure that is detachably attached to between the extractor and the disposal line and performs gas inspection of an inspection target gas extracted from the combustible gas facility.

According to this configuration, the inspection target gas used for the gas inspection is discharged to the exhaust gas treatment facility. Therefore, according to the above configuration, the inspection target gas extracted for the gas inspection can be appropriately subjected to the disposal treatment.

Moreover, in the gas inspection facility according to the embodiment, the disposal line is connected to an exhaust gas treatment facility located at the liquefied gas carrier and an exhaust gas treatment facility located on land, and the disposal line includes: a connection line connected to the gas inspection structure; a ship branch line connecting the connection line and the exhaust gas treatment facility located at the liquefied gas carrier; and a land branch line connecting the connection line and the exhaust gas treatment facility located on the land.

According to this configuration, the inspection target gas extracted for the gas inspection can be discharged to the exhaust gas treatment facility located at the liquefied gas carrier and the exhaust gas treatment facility located on land. Therefore, the degree of freedom of the disposal treatment of the inspection target gas improves.

Moreover, the gas inspection facility according to the embodiment can further include an inert gas supply structure that is detachably attached to between the extractor and the gas inspection structure and supplies an inert gas to between the extractor and the gas inspection structure.

According to this configuration, the inert gas can be easily supplied to the gas inspection line.

Moreover, in the gas inspection facility according to the embodiment, the gas inspection structure includes: an inlet line into which the inspection target gas flows; an outlet line from which the inspection target gas flows out; a main line connecting the inlet line and the outlet line; a gas inspector that is located on the main line and inspects the inspection target gas; a bypass line that connects the inlet line and the outlet line and is located in parallel with the main line; a switching valve that selects any of the main line and the bypass line as a line to which the inspection target gas having flowed through the inlet line is supplied; and a flow rate adjustment valve that is located on the inlet line and adjusts a flow rate of the inspection target gas in the inlet line.

According to this configuration, after the flow rate of the inspection target gas is adjusted, the inspection target gas can be supplied to the gas inspector. Therefore, the damage of the gas inspector which is caused when a large amount of inspection target gas flows into the gas inspector can be prevented.

Moreover, in the gas inspection facility according to Modified Example, the gas inspection structure includes a sampling balloon which is located upstream of the flow rate adjustment valve of the inlet line, temporarily stores the inspection target gas, and discharges the inspection target gas.

According to this configuration, the gas inspection can be performed in a state where the introduction of the inspection target gas from the combustible gas facility to the gas inspection line is stopped. Therefore, according to this configuration, the degree of freedom of the gas inspection improves.

### Reference Signs List

- 10: extractor
- 20: disposal line
- 21: connection line
- 22: ship branch line
- 23: land branch line
- 30: gas inspection structure
- 32: inlet line
- 33: outlet line
- 34: main line
- 35: bypass line
- 37: gas inspector
- 39: flow rate adjustment valve
- 40: switching valve
- 45: sampling balloon
- 50: inert gas supply structure
- 70: gas inspection line
- 100: gas inspection facility
- 101: liquefied gas carrier
- 102: combustible gas facility
- 110: ship exhaust gas treatment facility (exhaust gas treatment facility)
- 111: land exhaust gas treatment facility (exhaust gas treatment facility)
- 200: gas inspection facility

## Claims

1. A gas inspection method performed in a liquefied gas carrier,
the gas inspection method comprising:
attaching a gas inspection structure including a gas inspector to between an extractor located at a combustible gas facility and a disposal line connected to an exhaust gas treatment facility, to form a gas inspection line between the combustible gas facility and the exhaust gas treatment facility;
introducing an inspection target gas from the combustible gas facility to the gas inspection line;
subjecting the inspection target gas, introduced to the gas inspection line, to gas inspection by the gas inspector in the gas inspection line;
after the inspection target gas is subjected to the gas inspection, introducing an inert gas to the gas inspection line to discharge the inspection target gas, remaining in the gas inspection line, to the exhaust gas treatment facility through the disposal line; and
after an inside of the gas inspection line is deactivated, detaching the gas inspection structure from between the extractor and the disposal line.

2. The gas inspection method according to claim 1, wherein:
when introducing the inert gas to the gas inspection line, a percentage of a combustible gas in the gas in the gas inspection line is inspected by utilizing the gas inspector; and
the inert gas is introduced to the gas inspection line until the percentage of the combustible gas in the gas in the gas inspection line becomes a fixed percentage or less.

3. A gas inspection facility located at a liquefied gas carrier,
the gas inspection facility comprising:
an extractor located at a combustible gas facility;
a disposal line connected to an exhaust gas treatment facility; and
a gas inspection structure that is detachably attached to between the extractor and the disposal line and performs gas inspection of an inspection target gas extracted from the combustible gas facility.

4. The gas inspection facility according to claim 3, wherein:
the disposal line is connected to an exhaust gas treatment facility located at the liquefied gas carrier and an exhaust gas treatment facility located on land; and
the disposal line includes
a connection line connected to the gas inspection structure,
a ship branch line connecting the connection line and the exhaust gas treatment facility located at the liquefied gas carrier, and
a land branch line connecting the connection line and the exhaust gas treatment facility located on the land.

5. The gas inspection facility according to claim 3 or 4, further comprising an inert gas supply structure that is detachably attached to between the extractor and the gas inspection structure and supplies an inert gas to between the extractor and the gas inspection structure.

6. The gas inspection facility according to any one of claims 3 to 5, wherein the gas inspection structure includes:
an inlet line into which the inspection target gas flows;
an outlet line from which the inspection target gas flows out;
a main line connecting the inlet line and the outlet line;
a gas inspector that is located on the main line and inspects the inspection target gas;
a bypass line that connects the inlet line and the outlet line and is located in parallel with the main line;
a switching valve that selects any of the main line and the bypass line as a line to which the inspection target gas having flowed through the inlet line is supplied; and
a flow rate adjustment valve that is located on the inlet line and adjusts a flow rate of the inspection target gas in the inlet line.

7. The gas inspection facility according to claim 6, wherein the gas inspection structure includes a sampling balloon which is located upstream of the flow rate adjustment valve of the inlet line, temporarily stores the inspection target gas, and discharges the inspection target gas.
